# EUROPEAN PATENT APPLICATION

(11) **EP 3 915 671 A1**
(43) Date of publication of application: **01.12.2021**
(21) Application number: 20177225.8
(22) Date of filing: 28.05.2020
(51) Int. Cl.: B01F 3/04, C12M 1/00

(54) **SPARGING DEVICE**

(71) Applicant: Sartorius Stedim Biotech GmbH, 37079 Göttingen (DE)
(72) Inventor: Bode, Matthias, 37079 Göttingen (DE); Bode, Jonas, 37079 Göttingen (DE); Grimm, Christian, 37079 Göttingen (DE); Scholz, Jochen, 37079 Göttingen (DE); Prediger, Andreas, 37079 Göttingen (DE); Höhse, Marek, 37079 Göttingen (DE)
(74) Representative: Prinz & Partner mbB

(57) **Abstract**

A sparging device (10), especially for use in a bioprocess, comprises a medium chamber (20) and at least one gas chamber (24). The gas chamber (24) at least partially surrounds the medium chamber (20), or the medium chamber (20) at least partially surrounds the gas chamber (24). The medium chamber (20) and the gas chamber (24) are separated from each other by a wall (22). The wall (22) has a plurality of through-holes (32) or is composed of a porous material, such as a membrane or a porous ceramic. The sparging device (10) further comprises at least one gas inlet port (28) opening into the gas chamber (24) for inflow of gas.

## Description

The invention relates to a sparging device, especially for use in a bioprocess.

Spargers can be used to introduce air or other gases into a liquid medium. In the biotechnological context of cell cultivation, sparging controls the air or gas flow into the nutrient broth to create an optimal environment for cell growth. Accordingly, known spargers used in bioprocesses are specially designed for arrangement in bioreactors.

It is an object of the invention to provide a sparger design which is suitable for other applications and/or arrangements in a bioprocess, especially for arrangement in a tubing.

The above object is achieved by a sparging device according to claim 1. Advantageous and expedient embodiments of the invention are apparent from the dependent claims.

The invention provides a sparging device, especially for use in a bioprocess, comprising a medium chamber and at least one gas chamber. The gas chamber at least partially surrounds the medium chamber, or the medium chamber at least partially surrounds the gas chamber. The medium chamber and the gas chamber are separated from each other by a wall. The wall has a plurality of through-holes or is composed of a porous material, such as a membrane or a porous ceramic. The sparging device further comprises at least one gas inlet port opening into the gas chamber for inflow of gas.

The invention is based on the finding that the possibility of introducing gas into a tubing opens up completely new opportunities in the design of bioprocesses. It is then possible to sparge a medium, e.g. with O₂, CO₂ or N₂, at any location in a process where the medium passes through a tubing (hose, pipe etc.).

The sparging device according to the invention follows a new approach. Instead of providing only a perforated pipe or a similar structure which is to be inserted into or attached inside a bioreactor, the sparging device according to the invention itself provides a chamber for the liquid medium to be sparged. This medium chamber is at least partially surrounded by a gas chamber, or vice versa. From the gas chamber the gas can be introduced into the medium chamber through a separating wall via through-holes provided therein or due to the nature of the porous material of the separating wall. The medium chamber can be inserted into a flow path of the medium, especially into a tubing, or it can serve as a stand-alone bioreactor or reservoir.

The preferred application however is sparging a liquid medium while it flows through a tubing. In view of this use case, a basically cylindrically shaped medium chamber is advantageous. Thus, the sparging device can be designed as a rigid pipe piece for integration into the tubing.

In order to allow the liquid medium to constantly flow through the medium chamber, the medium chamber has a medium inlet opening and a medium outlet opening. For stop-flow or other applications in which the medium shall temporarily reside in the medium chamber, it may be expedient to provide at least one valve or other means directly at the medium inlet and/or outlet openings or in the adjoining tubing sections for selectively blocking and unblocking the medium flow.

In order to enable an easy fluid connection of the sparging device to the tubing into which the sparging device is to be integrated, an inlet connecting piece is formed at the medium inlet opening and an outlet connecting piece is formed at the medium outlet opening. Tri-clamp flange ends or hose barbs are preferred, as they allow for types of connection to adjoining tubing ends which are reliable and well established in bioprocess flow paths.

In a preferred embodiment, the medium inlet and outlet openings are located at different, preferably opposite ends of the medium chamber, and the at least one gas inlet port is located between the different ends. Thus, a very compact design of the sparging device can be achieved.

According to a preferred T-piece design of the sparging device, the medium inlet opening and the medium outlet opening define a medium flow direction within the medium chamber, the medium flow direction being oriented transversely relative to a gas inflow direction defined by the at least one gas inlet port. The angle between the medium flow direction and the gas inflow direction does not necessarily have to be around 90°, but may vary between 30° and 150°, for example.

In embodiments with the separating wall having through-holes, a preferred diameter of the through-holes is in the range of approximately 0.15 to 0.8 mm. This size of the through-holes has proven to be suitable for many applications of the sparging device, especially when it is inserted into a tubing.

The through-holes can be covered by a membrane cover. Depending on the structure and pore size of the membrane cover, it is thus possible to prevent that medium or suspended solids penetrate from the medium chamber into the gas chamber and into the connected line, which can be a pressure line. Further, the membrane cover can ensure sterility of the medium chamber, and it can serve to increase process safety and reliability.

For these purposes it is expedient to arrange the membrane cover in the gas chamber, rather than in the medium chamber.

Generally, details of the design of the sparging device can be varied in a lot of ways due to modern manufacturing techniques, such as, but not limited to, additive manufacturing (also known as 3D printing, including 3D bioprinting).

According to one design aspect, the density of through-holes in the inner wall can be varied along the flow direction to achieve a specific sparging behavior along the length of the device. For example, the through-holes may be arranged such that the sparging volume is kept constant or increases linearly over the length. Specific sparging behaviors can also be achieved by varying the outer diameter or the general shape of the gas chamber, for example.

According to another design aspect, the sparging device comprises at least one further gas chamber at least partially surrounding the medium chamber and being separated from the medium chamber by the wall, and at least one further gas inlet port opening into the further gas chamber. With such a design it is possible to introduce different gases into the liquid medium simultaneously or successively.

In a preferred embodiment of the sparging device, the gas chambers are helically wound around the medium chamber. It is thus possible to homogeneously introduce different gases into the medium at the same spot, i.e. over a defined axial portion of the medium chamber, at the same time. Further, such a design provides improved mechanical stability of the sparging device.

The at least one gas inlet port of the sparging device may be a pressure port and simply be connected to a compressed gas source with a pressure regulator. According to another aspect, a nozzle can be inserted into the gas inlet port. With specific nozzle designs (diameter, straight or bent nozzle, etc.) it is possible to achieve certain gas flow rates and bubble types.

Similar to the medium inlet and outlet openings, also the gas inlet port can be equipped with a connecting piece, preferably a tri-clamp flange end, to enable an easy connection to a line, which can be a pressure line, or a nozzle.

If the sparging device according to the invention is to be implemented in a multi-use bioprocess equipment, it is preferably made of stainless steel. It is also possible to produce the sparging device as a single-use item, though. In this case, the sparging device is made of a material suitable for sterilization by gamma radiation or in an autoclave. Some non-limiting examples of such materials are polypropylene (PP), high-density polyethylene (HDPE) and polybutylene terephthalate (PBT). It is advantageous to sterilize the sparging device already before shipment so that the user does not have any effort of sterilizing the device before integrating it into a process set-up.

The invention also relates to the use of a sparging device as defined above in a bioprocess. In particular, the sparging device can be used in a tubular bioreactor system or in a perfusion system, where it can be inserted in a filtration line, for example.

Further features and advantages of the invention will become apparent from the following description and from the accompanying drawings to which reference is made. In the drawings:
- Figure 1 shows a perspective view of a first embodiment of a sparging device according to the invention;
- Figure 2 shows a longitudinal section view of the sparging device of Figure 1;
- Figure 3 shows a perspective view of a second embodiment of a sparging device according to the invention;
- Figure 4 shows a longitudinal section view of the sparging device of Figure 3;
- Figure 5 shows a first cross section view of the sparging device of Figure 3 along line V-V in Figure 4;
- Figure 6 shows a second cross section view of the sparging device of Figure 3 along line VI-VI in Figure 4;
- Figure 7 shows the components of two variants of a third embodiment of a sparging device according to the invention; and
- Figure 8 shows the sparging device of Figure 7 in the assembled state.

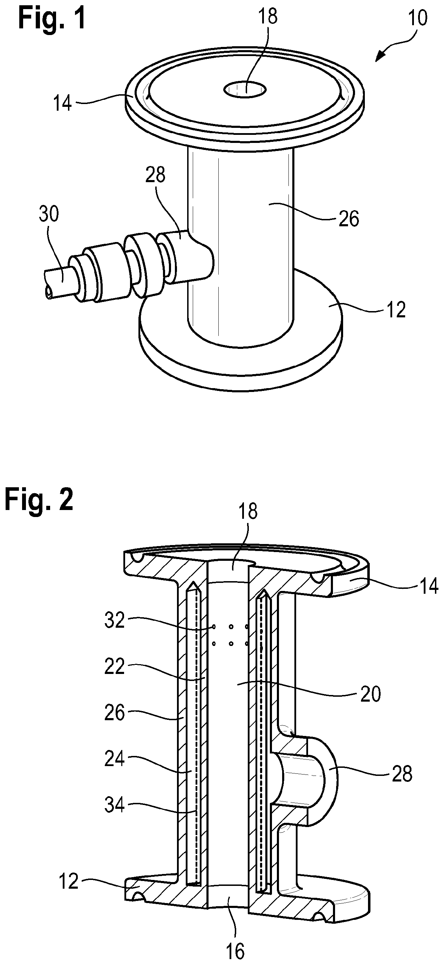

In Figures 1 and 2 a first embodiment of a sparging device 10 is shown. The sparging device has a generally cylindrical shape with fluid connecting pieces 12, 14 at the axial ends. The connecting pieces 12, 14, which are formed here as tri-clamp flange ends, but could also be hose barbs, for example, surround a medium inlet opening 16 and a medium outlet opening 18, respectively. The openings 16, 18 open into opposite ends of a medium chamber 20 which is confined by a cylindrical inner wall 22. The medium chamber 20 is surrounded by an annular gas chamber 24 which is confined by a cylindrical outer wall 26. Thus, the sparging device 10 is basically formed as a double-walled pipe piece, but the connecting pieces 12, 14 only allow access to the medium chamber 20, i.e. the axial ends of the gas chamber 24 are closed.

In the outer wall 26 of the sparging device 10 a gas inlet port 28 is formed for connecting a line 30, which can be a pressure line (compressed gas line). The gas inlet port 28 is located between the axial ends of the sparging device 10 in a perpendicular orientation relative to the longitudinal axis of the medium chamber 20 and the gas chamber 24. The gas inlet port 28 opens into the gas chamber 24 which is separated from the medium chamber 20 by the inner wall 22.

A plurality of through-holes 32 is formed in the inner wall 22 at pre-defined locations in a pre-defined pattern. The through-holes 32 allow the gas supplied from line 30 to the gas chamber 24 via the gas inlet port 28 to enter the medium chamber 20 in a defined manner. The through-holes 32 have a preferred diameter in the range of approximately 0.15 to 0.8 mm. Within the gas chamber 24 a membrane cover 34 covering the through-holes 32 may be arranged.

The sparging device 10 shown in Figures 1 and 2 is designed as a single-use component. It is made of a material suitable for sterilization by gamma radiation or in an autoclave, such as polypropylene (PP), high-density polyethylene (HDPE) or polybutylene terephthalate (PBT), for example. 3D printing, molding or other additive manufacturing techniques (also known as 3D printing, including 3D bioprinting) can be used to build the sparging device 10.

In operation, the sparging device 10 is integrated into a tubing of a bioprocess set-up. The medium inlet and outlet openings 16, 18 are connected to tubing ends via the connecting pieces 12, 14. A liquid medium of the bioprocess flows through the tubing and through the medium chamber 20 of the sparging device 10. At the same time, gas from a gas source, which can be a compressed gas source, is supplied via line 30 and the gas inlet port 28 to the gas chamber 24. According to the design of the embodiment, the gas inflow direction is perpendicular relative to the direction of the medium flowing through the medium chamber 20. The supplied gas is introduced into the medium in the medium chamber 20 in a controlled manner via the through-holes 32 of the inner wall 22.

The membrane cover 34, on the one hand, protects line 30 from medium that may enter from the medium chamber 20 through the through-holes 32 into the gas chamber 24. On the other hand, the membrane cover 34 acts as a sterile barrier, thus keeping the medium chamber 20 free from external contamination.

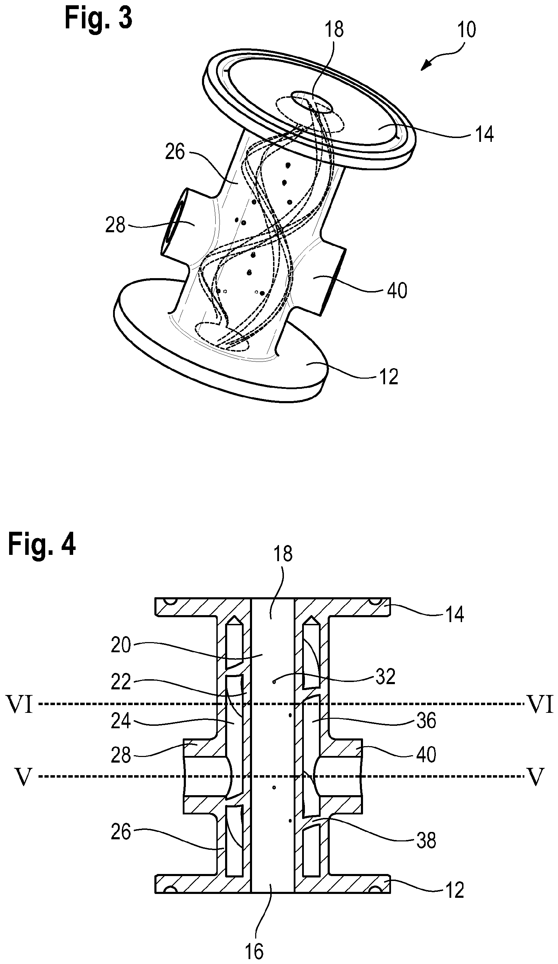

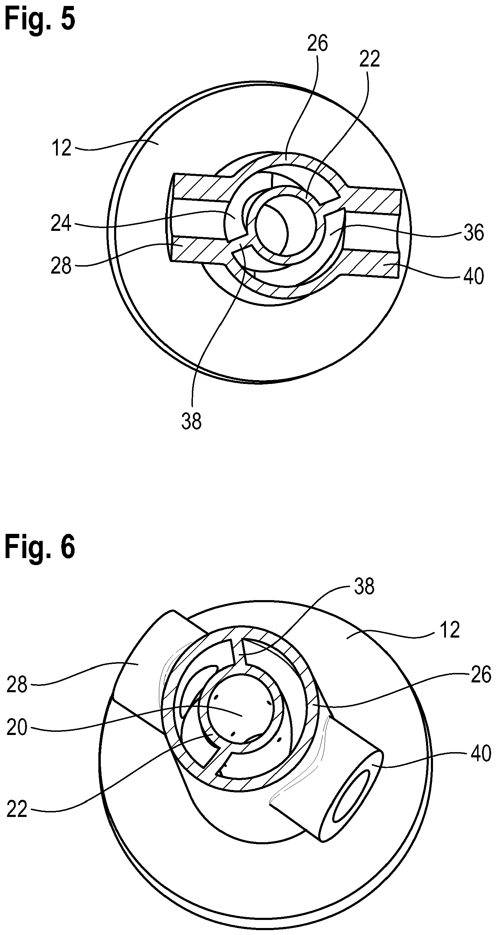

Figures 3 to 6 show a second embodiment of the sparging device 10 which is very similar to the first embodiment described above. The major difference is that the sparging device 10 has two separate gas chambers 24, 36 extending in the space between the inner wall 22 and the outer wall 26. The gas chambers 24, 36 are separated from each other by intermediate wall sections 38 and have their own gas inlet ports 28, 40, respectively.

From the sectional views of Figures 4, 5 and 6 it is apparent that due to the helical design of the intermediate wall sections 38 both gas chambers 24, 36 partially surround the medium chamber 20. In fact, both gas chambers 24, 26 are helically wound around the medium chamber 20 next to each other. Both gas chambers 24, 36 are fluidly connected to the medium chamber 20 via a set of through-holes 32. Thus, two different gases can be introduced into the medium in the medium chamber 20 via the two separate gas chambers 24, 36.

In a similar manner, embodiments with even more than two gas chambers can be provided.

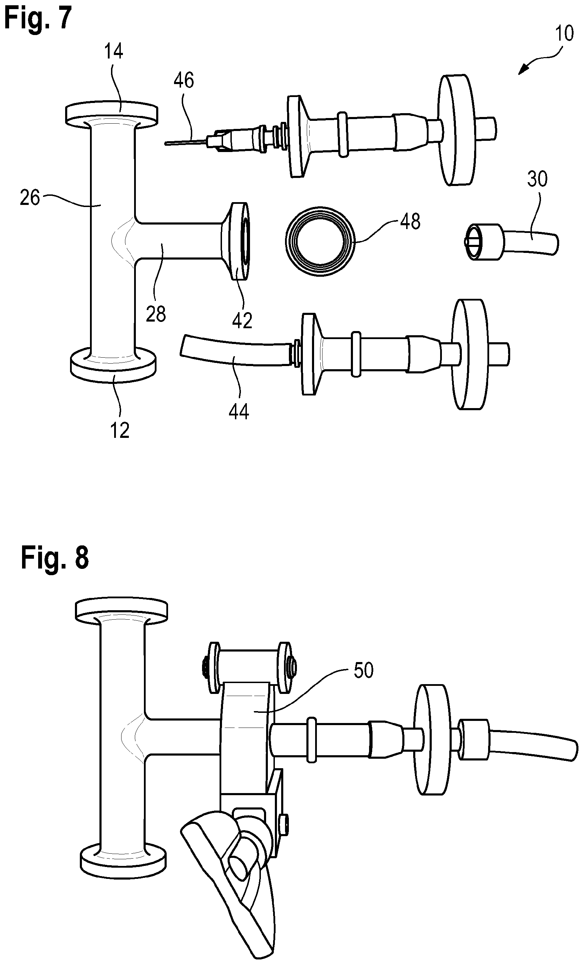

A third embodiment of the sparging device 10 is shown in Figures 7 and 8. Figure 7 shows all individual components of two different variants of the sparging device 10 separately, whereas Figure 8 shows the sparging device 10 in the assembled state.

The main body of the sparging device 10, including the inner wall 22 (not visible) and the outer wall 26 defining the medium chamber 20 and the gas chamber 24, the gas inlet port 28 and the connecting pieces 12, 14 at the medium inlet and outlet openings 16, 18 as well as a further connecting piece 42 at the gas inlet port 28 (here all formed as tri-clamp flange ends), is made of stainless steel. Thus, the sparging device 10 is configured as a multi-use device.

According to a first variant, the sparging device 10 further includes a hose piece 44 for insertion into the gas inlet port 28. In this specific variant, the gas inlet port 28 is a pressure port. The hose piece 44 is connected to a line 30, which is a pressure line here, with optional further components arranged in between. According to a second variant, the hose piece 44 is replaced by a nozzle 46.

In both variants, a seal 48 and a matching clamp 50 are used to connect the hose piece 44 or the nozzle 46 to the tri-clamp flange end at the gas inlet port 28.

The basic operating principle of the third embodiment of the sparging device 10 is similar to that of the first embodiment. Of course, it is possible to provide also a multi-use sparging device 10 having further gas chambers and one or more gas inlet ports.

While in the above-described embodiments the medium chamber 20 and the gas chamber(s) 24, 36 are separated from each other by a wall 22 having a plurality of through-holes 32, the wall 22 can also be composed of a porous material, such as a membrane or a porous ceramic, in other embodiments.

Further, a reversed arrangement of the medium chamber 20 and the gas chamber(s) 24, 36 is possible. In this case, the gas chamber(s) 24, 36 are surrounded by an annular medium chamber 20.

In general, the embodiments described above are to be understood as non-limiting examples. Thus, certain features of different embodiments can be combined in a suitable manner. Moreover, other designs with non-cylindrical medium chambers and gas chambers are also possible. For example, the medium chamber may be configured as a vessel for fermentation or as a reservoir of a liquid medium.

### List of Reference Signs

- 10: sparging device
- 12: inlet connecting piece
- 14: outlet connecting piece
- 16: inlet opening
- 18: outlet opening
- 20: medium chamber
- 22: inner wall
- 24: gas chamber
- 26: outer wall
- 28: gas inlet port
- 30: line
- 32: through-holes
- 34: membrane cover
- 36: further gas chamber
- 38: intermediate wall sections
- 40: further gas inlet port
- 42: further connecting piece
- 44: hose piece
- 46: nozzle
- 48: seal
- 50: clamp

## Claims

1. A sparging device (10), comprising a medium chamber (20) and at least one gas chamber (24), the gas chamber (24) at least partially surrounding the medium chamber (20), or the medium chamber (20) at least partially surrounding the gas chamber (24), the medium chamber (20) and the gas chamber (24) being separated from each other by a wall (22), the wall (22) having a plurality of through-holes (32) or being composed of a porous material, the sparging device (10) further comprising at least one gas inlet port (28) opening into the gas chamber (24) for inflow of gas.

2. The sparging device (10) according to claim 1, **characterized in that** the medium chamber (20) basically is of cylindrical shape.

3. The sparging device (10) according to claim 1 or 2, **characterized in that** the medium chamber (20) has a medium inlet opening (16) and a medium outlet opening (18).

4. The sparging device (10) according to claim 3, **characterized in that** an inlet connecting piece (12) is formed at the medium inlet opening (16) and an outlet connecting piece (14) is formed at the medium outlet opening (16), the connecting pieces (12, 14) preferably being tri-clamp flange ends or hose barbs.

5. The sparging device (10) according to claim 3 or 4, **characterized in that** the medium inlet and outlet openings (16, 18) are located at different ends of the medium chamber (20), the gas inlet port (28) being located between the different ends.

6. The sparging device (10) according to any of claims 3 to 5, **characterized in that** the medium inlet opening (16) and the medium outlet opening (18) define a medium flow direction within the medium chamber (20), the medium flow direction being oriented transversely relative to a gas inflow direction defined by the gas inlet port (28).

7. The sparging device (10) according to any of the preceding claims, **characterized in that** the through-holes (32) have a diameter in the range of approximately 0.15 to 0.18 mm.

8. The sparging device (10) according to any of the preceding claims, **characterized in that** the through-holes (32) are covered by a membrane cover (34).

9. The sparging device (10) according to claim 8, **characterized in that** the membrane cover (34) is arranged in the gas chamber (24).

10. The sparging device (10) according to any of the preceding claims, **characterized in that** the density of through-holes (32) in the wall (22) varies along the flow direction.

11. The sparging device (10) according to any of the preceding claims, further comprising a nozzle inserted into the gas inlet port (28).

12. The sparging device (10) according to any of the preceding claims, **characterized in that** a pressure connecting piece is formed at the gas inlet port (28), the pressure connecting piece preferably being a tri-clamp flange end.

13. The sparging device (10) according to any of the preceding claims, further comprising at least one further gas chamber (36) at least partially surrounding the medium chamber (20) and being separated from the medium chamber (20) by the wall (22), and at least one further gas inlet port (40) opening into the further gas chamber (36).

14. The sparging device (10) according to claim 13, the gas chambers (24, 36) being helically wound around the medium chamber (20).

15. The sparging device (10) according to any of the preceding claims, **characterized in that** the device is made of a material suitable for sterilization by gamma radiation or in an autoclave, preferably including one of the following: polypropylene (PP), high-density polyethylene (HDPE), polybutylene terephthalate (PBT).

16. Use of a sparging device (10) according to any of the preceding claims in a bioprocess, especially in a tubular bioreactor system or in a perfusion system.
